# EUROPEAN PATENT SPECIFICATION

(11) **EP 0 576 899 B1**
(45) Date of publication and mention of the grant of the patent: **26.08.1998**
(21) Application number: 93109539.2
(22) Date of filing: 15.06.1993
(51) Int. Cl.: C12N 15/55, C12N 15/78, C12N 1/21, C12N 9/20

(54) **A gene coding a protein having a lipase activity and a process for producing the same**
Gen, das für ein Protein mit Lipase-Aktivität kodiert und Verfahren zur Herstellung
Gène codant pour une protéine ayant une activité lipasique et procédé de production

(30) Priority: 17.06.1992 JP 183093/92
(43) Date of publication of application: 05.01.1994
(73) Proprietor: CHISSO CORPORATION, Osaka (JP)
(72) Inventor: Aoyama, Shigeyuki, Yokohamashi, Kanagawaken (JP); Yoshida, Naoyuki, Ichiharashi, Chibaken (JP)
(74) Representative: Kraus, Walter, Dr.

(56) References cited:
- EP-A- 318 775
- EP-A- 331 376
- EP-A- 451 452
- Section Ch, Week 9320, 16 April 1993 Derwent Publications Ltd., London, GB; Class B04, AN 93-161729
- DATABASE WPI Section Ch, Week 9141, 29 August 1991 Derwent Publications Ltd., London, GB; Class B04, AN 91-299434

## Description

This invention relates to a gene coding a protein having a lipase activity originated from a bacterium of Pseudomonas genus, a recombinant DNA containing the gene, a bacterium of Pseudomonas genus containing the DNA and a process for producing a protein having a lipase activity, characterized by secretory production outside bacterial cells.

Lipases have been used as a lipid-hydrolyzing enzyme, for oil and fat processing, clinical diagnostic medicine, detergent, digestant, etc., and further, in recent years, lipases have been an important enzyme for catalyzing ester hydrolysis, ester synthesis or transesterificaton, as processes for producing chemical products, particularly, optically active compounds. Further, it has been known that bacteria of Pseudomonas genus produce a lipase useful as a catalyst for ester hydrolysis, ester synthesis or transesterification (see JP 62-166898).

EP-A-318 775 discloses a DNA arrangement coding a lipase originated from Pseudomonas genus bacterium, a recombinant DNA replicable inside Escherichia coli encoding said lipase, Escherichia coli transformed by a recombinant DNA encoding said lipase and a process for producing said lipase.

EP-A-0 451 452 discloses a recombinant plasmid for lipase expression comprising (i) a vector of a multicopy type replicative inside Escherichia coli, (ii) a fused promoter (tac promoter) of a promoter of Escherichia coli tryptophan operon with a promoter of Escherichia coli lactose operon and (iii) a DNA sequence coding a lipase originated from Pneudomonas fragi IFO 12049 strain under the rule of said fused promoter. Furthermore, EP-A-0 451 452 discloses an Escherichia coli strain transformed with the respective recombinant plasmid and a process for producing a lipase which comprises the use of said Escherichia coli strain.

In order to commercially utilize a lipase originated from bacterium of Pseudomonas genus and provided with such characteristics, production of an enzyme in a large quantity has been desired. Further, for its production on a commercial scale, secretory production of the enzyme into the culture solution has been regarded as effective in view of the advantages of continuous cultivation, easiness in the enzyme recovery, etc. In recent years, application of gene manipulation technique has been introduced into production of useful proteins in a large quantity, and this has also been applied also to lipases of bacteria of Pseudomonas genus.

However, in spite of such attempts, an efficient secretory production system of lipases into the culture solution has not yet been completed, and problems have been raised in respect of commercial utilization.

For example, when Escherichia coli is used as a host bacterium, recovery of the produced lipase has been carried out from the inside of the bacterial cells (JP 62-188072 and JP 62-228279 and JP 1-7757). In such a case of production inside the bacterial cells, the recovery of the produced substance requires a bacterium-breaking step such as supersonic treatment and a number of purification steps for removing impurities originated from host bacterium. Thus, such processes have been regarded as disadvantageous, in the aspect of production of such a substance.

Further, when Pseudomonas bacterium is used as a host bacterium, recovery of lipase has been carried out from the inside of the culture solution which is advantageous for commercial utilization (JP 63-300386 and JP 1-338250). However, in such cases, cultivation has been carried out e.g. for 3 days for the production. Thus, such production cannot always be regarded as efficient.

In solving the above-mentioned problems, the present inventors considered that when a strain producing a protein having a lipase activity originated from Pseudomonas genus bacterium, in a high extent secretion, is created according to a gene recobminant technique, it might be possible to produce the lipase on a commercial scale, and have made extensive research. As a result, it was found that when a DNA fragment containing a gene coding a protein having a lipase activity is isolated from a chromosome DNA of Pseudomonas genus bacterium, to obtain a recombinant DNA having the DNA fragment, then Pseudomonas genus bacterium transformed by the recombinant DNA secretorily produces a notably large quantity of a protein having a lipase activity in the culture solution, and have completed the present invention.

Namely, the object of the present invention is to provide a DNA fragment containing a gene coding a protein having a lipase activity originated from a bacterium of Pseudomonas genus, a recombinant DNA having the above DNA fragment, a bacterium of Pseudomonas genus suitable to efficient secretory production of a protein transformed by the above recombinant DNA and having a lipase activity, and a process for producing a protein having a lipase activity, using the above bacterium.

The present invention has the following constitutions:
(1) A gene coding a protein having a lipase activity originated from a bacterium of Pseudomonas genus, characterized by having the following base sequence: 
10 20 30 40 50 60
5'ATGAAACATT TAATACAGTT TGTTTTGCTA ATGTCTGCGC CCCAAACAAC AAAAACAGGT
70 80 90 100 110 120
CATCACATGC GCAGGCTCTC CCTACTCATC CCTTGGCGGG GATGCATCCT GTCCATCGTC
130 140 150 160 170 180
AGTGAACGTG CCATAGCAGC ACCCTCGCCG TATTCCACCA TGATCGTGTT CGGCGACAGC
190 200 210 220 230 240
CTGAGCGACT CGGGGCATTT TCCAGGGGTC GGCACAGGCA TGCGCTTCAC CAACCGCACC
250 260 270 280 290 300
GGGCCGACGT ACAGGGATTA CCGGGGGGAG GAATATGCTG CAGTAACGCC CACGCGGCTG
310 320 330 340 350 360
GGCACCCAAC TGGGGATCGC CCCCGCAGAC CTGCGCCCGT CCACTTCGCC GGGCAACTCT
370 380 390 400 410 420
 
CTTGCAGGGG AGCCCGACGG CAACAACTGG GCGGTCGGCG GCTATCGCAC AGACCAGATC
430 440 450 460 470 480
CTCAATTCCA TCAAGACCGA GTCAAAGGTC GCCATCCCGG ATGACTGGTC CTTCGTCGGC
490 500 510 520 530 540
GGCTACGTCC TGCGCAGCAA GCCTGGCTAC CTTGTGCAGA ACAGCTTCAC GGCTGACCCC
550 560 570 580 590 600
AAGGCGTTGT ATTTCATTTC AGGCGGCGGT AATGACTTTC TCCAAGGCAA GGTCACCAAC
610 620 630 640 650 660
CCCGCCGAGG CAGGCCAAGC CGCACAAAGG CTGGCGGCAA GCGCCCACAC CCTGCAACAG
670 680 690 700 710 720
GCAGGGGCAC GCTACATCAT GGTCTGGCTG CTGCCCGATC TGGGCCTGAC GCCAGCCGTT
730 740 750 760 770 780
TACGGTACAC CCACCCAGGC TGGCACCAGC GCCCTGAGCG CCCTGTTCAA TCATGAGCTA
790 800 810 820 830 840
ACCCAGCAAC TGGCGCAGAT AGACGCCGAA GTGATTCCCC TCAATATCCC GCTGCTGCTG
850 860 870 880 890 900
CGTGAAGCCA TCGCCGACCC CGCCCGTTAT GGCCTGGCAC TGGGGCAGGA CCTTGTCGCG
910 920 930 940 950 960
ACCTGTTTCA ATGGCGAAGA GTGTACGGAA AACCCGCAGT ACGGACTCAA CAGCGCAACC
970 980 990 1000 1010 1020
CCCAACCCGG CCAAACTGCT ATTCAACGAC TCGGTACACC CCACCGAGAC AGGGCAACAG
1030 1040 1050 1060 1070 1080
CTGATTGCCG ACTATGCCTA CTCATTGCTG GCCGTGCCGT GGGAGCTGAC CTTGTTACCC
1090 1100 1110 1120 1130 1140
GTTATGGCCC AAGGCTCGCT CAACGCCCAT CAAGACCAGT TGCGCAACCA ATGGGCTGGC
1150 1160 1170 1180 1190 1200
GACGACGGTC AATGGCAAGC CATTGGCCAG TGGCGAACCC TGCTCGCCGG TGGCGGGCAA
1210 1220 1230 1240 1250 1260
CGCCTGGAAA TCGACAAGCA GACCACGGCC GTGAAGGCAG ATGGCAAAGG CTATAACCTC
 
1270 1280 1290 1300 1310 1320
AATATCGGCA CCAGCTACAG GCTGGATGAC AACTGGCGAT TTGGCATTGC CGGCGGGTTT
1330 1340 1350 1360 1370 1380
TACCGGCAAC GCCTGGAAAC CGGGGCCAAT AAATCGGACT ACAAGCTCAA CAGCTATCTG
1390 1400 1410 1420 1430 1440
GGTAGCGTGT TTGCCCAATA CCAGCACAAT CACTGGTGGG GGGATGCGGC ACTGACGTTG
1450 1460 1470 1480 1490 1500
GGCAGGCTGG ATTACGACAG CCTGAAACGA AAATTCGCGC TTGGGGTGGG TAGTGATATG
1510 1520 1530 1540 1550 1560
GAGCAAGGCC AGGCTGACGG CCATTTGCGA GCATTGGTAC ACGCCTGGGT TATGAAATCG
1570 1580 1590 1600 1610 1620
CACAGGCATC TGACCTGTGG CAGCTCTCCC CCTTTATCAG CGCCGTTACT CGCGGGTGGA
AGTTAA 3'
(2) A gene according to item (1) wherein said bacterium of Pseudomonas genus is Pseuodmonas fragi IFO 120 49 strain.
(3) A recombinant DNA capable of being replicated in a bacterium of Pseudomonas genus and containing the gene of items 1 and 2, incorporated into a broad host range vector of Gram-negative bacteria.
(4) A bacterium of Pseudomonas genus transformed by a recombinant DNA according to item (3).
(5) A process for producing a protein having a lipase activity, which process comprises cultivating a bacterium of Pseudomonas genus transformed by a recombinant DNA according to item (4), to thereby produce a protein having a lipase activity in the culture medium, and recovering this protein from the culture medium.

Fig. 1 shows an example of a restriction enzyme map of Eco RI 3.7 Kb fragment having a plasmid pCCB inserted thereinto and the results of a deletion experiment wherein the presence or absence of clear zone formation is made an indication. The symbol (+) shows a clear zone formation.

Fig. 2 shows a construction figure of the expression plasmid pUC-RSFKmCCB of a protein having a lipase activity.

The present invention will be described in more detail.

### ① Cloning of a gene coding a protein having a lipase activity:

The chromosome DNA of Pseudomonas fragi IFO 12049 strain can be prepared for exmaple according to Smith's method (Smith, M.G., Methods in Enzymology, Academic press, New York, 12, part A, p. 545, (1967)).

Incorporation of a chromosome DNA into a vector DNA can be carried out by digesting the chromosome DNA and the vector DNA with a restriction enzyme, followed by mixing the two and treating the mixture with a DNA lipase.

Examples of the restriction enzyme are EcoRI, BamHI, Sau3AI. As the vector DNA, known ones such as pUC9, λgt10, Charomid DNA are utilizable. The thus obtained recombinant DNA can transform Escherichia coli according to a known method such as CaCℓ₂ method, in vitro packaging method, etc.

Choice of a transformed strain having a lipase activity is carried out by isolating a strain forming a clear zone accompanying the hydrolysis of tributyrin on a tributyrin-agar medium. A recombinant DNA containing a gene coding a protein having a lipase activity can be obtained from the above obtained transformed strain, according to a known technique such as alkali method.

Next, the region necessary for the expression of the protein having a lipase activity in the transformed bacterium can be determined by subcloning the above recombinant DNA using various restriction enzymes, and observing the clear zone-formability of such transformed strain on the tributyrin-agar medium. Further, the gene coding the protein having a lipase activity and the base sequence in the region necessary for the expression can be determined according to a known technique such as dideoxy method (Sanger.F., et al, Proc. Natl. Acad. Sci. U.S.A., 74, 5463 (1977)).

### ②Production of the protein having a lipase activity:

When a DNA fragment containing a gene coding the protein obtained in the above manner is introduced into a plasmid vector which is replicative inside the bacterium of Pseudomonas genus, according to a known conjunction-transmission method or the like, it is possible to construct a recombinant DNA for lipase expression.

As such a plasmid vector, for example, a broad range host vector RSF1010, those obtained by introducing a suitable selective marker such as kanamycin-resistant gene into RSF1010 are usable.

Further, by cultivating a bacterium of Pseudomonas genus transformed by the recombinant DNA obtained in the above manner, under optimum conditions for enzyme production, a protein having a lipase activity is produced. Further, by measuring the lipase activity in the culture solution, it is possible to obtain an optimum host bacterium of Pseudomonas genus for secretorily expressing a protein having a lipase activity. As the thus obtained bacterium of Pseudomonas genus, for example, Pseudomonas putida AC10 strain and the like are usable.

Further, the measurement of lipase activity can be carried out using a commercially available, lipase activity-measuring kit, or the like. Pseudomonas putida AC10 strain having a recombinant DNA obtained according to the present invention introduced thereinto is cultivated under optimum conditions for enzyme production, and a protein having a lipase activity is separated from the culture medium and purified.

The present invention will be described in more detail by way of Example.

### Examples

### (1) Preparation of chromosome DNA:

Pseudomonas fragi IFO 12049 strain was cultivated in an LB medium (1% bactotripton, 0.5% yeast extract and 0.5% NaCℓ), at 30°C overnight. The bacterial cells were collected, followed by washing and suspending the cells in a solution of 50 mM Tris-Hcℓ buffer
(pH 8.0) and 10 mM EDTA (12 mℓ). Lysozyme (1 mg) was added to the resulting suspension, followed by allowing the mixture to stand at room temperature for one hour, adding to the resulting solution, Protenase K (trademark of product made by Merck Co., Ltd.) so as to give a concentration of 100 µg/mℓ, in the presence of 0.5% SDS, slowly shaking the mixture at 55°C for 3 hours, extracting this resulting sample with phenol and precipitating it with ethanol to prepare chromosome DNA.

### (2) Cloning of a gene coding a protein having a lipase activity and analysis of the gene:

The thus prepared chromosome DNA was digested with a restriction enzyme EcoRI, followed by subjecting the resulting substance to electrophoresis in an agarose gel to recover fragments of 2 to 4 Kb, linking the recovered DNA fragment to EcoRI site of Charomid 9-42 (trademark of product made by Nippon Gene Co., Ltd.) with a DNA lipase, and infecting the linked DNA in the form of phage with Escherichia coli VCS 257 using λDNA in vitro packaging kit (Gigapack IIPLUS (tradename of product made by STRATAGENE Co., Ltd.)) to effect transduction.

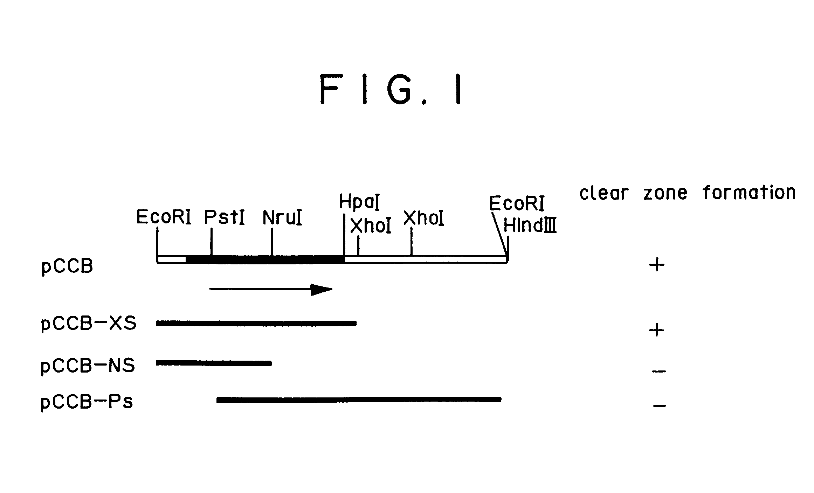

The resulting transformed strain was subjected to plating on an LB medium containing ampicillin (50 µg/mℓ) and 1% tributyrin, at 37°C for 24 hours to obtain two clear zone-forming strains from among about 1,100 transformed strains, extracting a plasmid from the above two clear zone-forming strains, digesting EcoRI, analyzing inserted DNA fragments by agarose gel electrophoresis to confirm that both the strains contained about 3.7 Kb EcoRI fragment, and subcloning the 3.7 Kb EcoRI DNA fragment at the EcoRI site of pTV 119 N (made by Takara Shuzo Co., Ltd.), to prepare a plasmid pCCB. Fig. 1 shows a restriction enzyme map of 3.7 Kb DNA fragments having EcoRI inserted at pCCB. Next, the 3.7 Kb EcoRI fragments of the plasmid pCCB were broken by various kinds of restriction enzymes, followed by subcloning the respective fragments at pTV 119N to transformm Escherichia coli MV 1184 strain, and observing the presence or absence of clear zone formation on the tributyrin-containing LB medium, in the same manner as above.

As a result, a clear zone formation was observed in Escherichia coli having a plasmid pCCB-XS having EcoRI-XhoI 2.1 Kb fragment, as shown in Fig. 1. As a result, it was suggested that a gene coding a protein having a lipase activity and a region necessary for its expression were present in EcoRI-XhoI 2.1 Kb fragment.

### (3) Determination of base sequence:

The base sequence of inserted EcoRI 3.7 Kb DNA fragment of the plasmid pCCB was determined according to dideoxy method (F. Sanger et al, Proc. Natl. Acad. Sci. U.S.A., 74, 5463 (1977)). As a result, an open reading frame was present within a region shown by an arrow mark (→) in Fig. 1.

The base sequence of the gene and the corresponding amino acid sequence table are shown at the end of this section.

### (4) Production of a protein having a lipase activity:

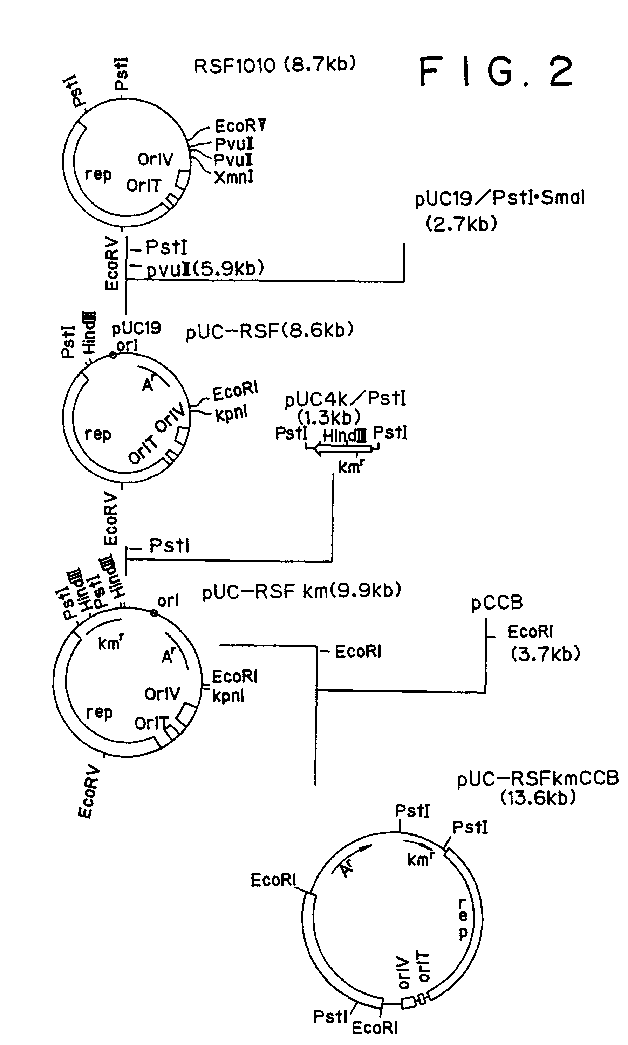

An expression plasmid pUC-RSFKmCCB was constructed according to the steps shown in Fig. 2.

To 5.9 Kb fragment obtained by digesting PstI and PvuII of a broad range host vector RSF1010 was linked 2.7 Kb fragment obtained by digesting PstI and SmaI of pUC19 to obtain a plasmid pUC-RSF, followed by introducing into PstI site of this plasmid, 1.3 Kb fragment of PstI containing a kanamycin-resistant gene, and introducing into the EcoRI site of the resulting plasmid, pUC-RSFkm, EcoRI 3.7 Kb fragment of pCCB, to construct the expression plasmid pUC-RSFKmCCB.

This plasmid pUC-RSFKmCCB was introduced into Pseudomonas putida AC10 strain, according to Simon et al's conjugation method (R. Simon et al, Biotechnology, 1.784 (1983)). This Pseudomonas putida AC10 (pUC-RSFKmCCB) strain was cultivated on an LB medium containing ampicillin and kanamycin (each, 50 µg/mℓ), at 30°C overnight. The lipase activity of the filtrate of the culture medium was measured using Lipase Kit S (tradename of product made by Dainippon Pharmaceutical Co., Ltd.) to give 268 BALB unit per 100 µℓ of the filtrate.

The results of measurements of Pseudomonas fragi IFO 12049 strain and Pseudomonas putida AC 10 strain, carried out under the same conditions were both, values lower than the lower limit of the measurement sensitivity (50 BALB unit) per 100 µℓ of the filtrate. In addition, the measurement method and the calculation method of measurement unit were carried out according to the method of Lipase Kit S.

In the above Example, plasmid preparation from bacterial cells, the reaction using restriction enzymes, etc. and treatment of DNA fragment were carried out in a conventional manner unless otherwise indicated. In addition, as to the restriction enzymes, DNA ligase, etc., those prepared by Takara Shuzo Co., Ltd. and Japan Gene Co., Ltd. were used.

### SEQUENCE LISTING

### GENERAL INFORMATION

### APPLICANT:

NAME: CHISSO CORPORATION
STREET: 6-32, NAKANOSHIMA 3-CHOME, KITAKU
CITY: OSAKA
COUNTRY: JAPAN

### TITLE OF INVENTION:

A GENE CODING A PROTEIN HAVING A LIPASE ACTIVITY AND A PROCESS FOR PRODUCING THE SAME

### NUMBER OF SEQUENCES: 1

COMPUTER READABLE FORM: MEDIUM TYPE: DISKETTE
COMPUTER/OPERATING SYSTEM: PC-9801/NEC
SOFTWARE: WORDPERFECT

### INFORMATION FOR SEQ ID NO. 1:

### SEQUENCE CHARACTERISTICS:

LENGTH OF SEQUENCE: 1,626
TYPE OF SEQUENCE: NUCLEIC ACID
NUMBER OF CHAIN: TWO
TOPOLOGY: LINEAR CHAIN
KIND OF SEQUENCE: GENOMIC DNA
HYPOTHETICAL: YES

### ORIGIN

NAME OF LIVING: PSEUDOMONAS FRAGI
NAME OF STRAIN: IFO 12049

ATG AAA CAT TTA ATA CAG TTT GTT TTG CTA ATG TCT GCG CCC CAA ACA
Met Lys His Leu Ile Gln Phe Val Leu Leu Met Ser Ala Pro Gln Thr

ACA AAA ACA GGT CAT CAC ATG CGC AGG CTC TCC CTA CTC ATC CCT TGG
Thr Lys Thr Gly His His Met Arg Arg Leu Ser Leu Leu Ile Pro Trp

CGG GGA TGC ATC CTG TCC ATC GTC AGT GAA CGT GCC ATA GCA GCA CCC
Arg Gly Cys Ile Leu Ser Ile Val Ser Glu Arg Ala Ile Ala Ala Pro

TCG CCG TAT TCC ACC ATG ATC GTG TTC GGC GAC AGC CTG AGC GAC TCG
Ser Pro Tyr Ser Thr Met Ile Val Phe Gly Asp Ser Leu Ser Asp Ser

GGG CAT TTT CCA GGG GTC GGC ACA GGC ATG CGC TTC ACC AAC CGC ACC
Gly His Phe Pro Gly Val Gly Thr Gly Met Arg Phe Thr Asn Arg Thr

GGG CCG ACG TAC AGG GAT TAC CGG GGG GAG GAA TAT GCT GCA GTA ACG
Gly Pro Thr Tyr Arg Asp Tyr Arg Gly Glu Glu Tyr Ala Ala Val Thr

CCC ACG CGG CTG GGC ACC CAA CTG GGG ATC GCC CCC GCA GAC CTG CGC
Pro Thr Arg Leu Gly Thr Gln Leu Gly Ile Ala Pro Ala Asp Leu Arg

CCG TCC ACT TCG CCG GGC AAC TCT CTT GCA GGG GAG CCC GAC GGC AAC
Pro Ser Thr Ser Pro Gly Asn Ser Leu Ala Gly Glu Pro Asp Gly Asn

AAC TGG GCG GTC GGC GGC TAT CGC ACA GAC CAG ATC CTC AAT TCC ATC
Asn Trp Ala Val Gly Gly Tyr Arg Thr Asp Gln Ile Leu Asn Ser Ile

AAG ACC GAG TCA AAG GTC GCC ATC CCG GAT GAC TGG TCC TTC GTC GGC
Lys Thr Glu Ser Lys Val Ala Ile Pro Asp Asp Trp Ser Phe Val Gly

GGC TAC GTC CTG CGC AGC AAG CCT GGC TAC CTT GTG CAG AAC AGC TTC
Gly Tyr Val Leu Arg Ser Lys Pro Gly Tyr Leu Val Gln Asn Ser Phe

ACG GCT GAC CCC AAG GCG TTG TAT TTC ATT TCA GGC GGC GGT AAT GAC
Thr Ala Asp Pro Lys Ala Leu Tyr Phe Ile Ser Gly Gly Gly Asn Asp

TTT CTC CAA GGC AAG GTC ACC AAC CCC GCC GAG GCA GGC CAA GCC GCA
Phe Leu Gln Gly Lys Val Thr Asn Pro Ala Glu Ala Gly Gln Ala Ala

CAA AGG CTG GCG GCA AGC GCC CAC ACC CTG CAA CAG GCA GGG GCA CGC
Gln Arg Leu Ala Ala Ser Ala His Thr Leu Gln Gln Ala Gly Ala Arg

TAC ATC ATG GTC TGG CTG CTG CCC GAT CTG GGC CTG ACG CCA GCC GTT
Tyr Ile Met Val Trp Leu Leu Pro Asp Leu Gly Leu Thr Pro Ala Val

TAC GGT ACA CCC ACC CAG GCT GGC ACC AGC GCC CTG AGC GCC CTG TTC
Tyr Gly Thr Pro Thr Gln Ala Gly Thr Ser Ala Leu Ser Ala Leu Phe

AAT CAT GAG CTA ACC CAG CAA CTG GCG CAG ATA GAC GCC GAA GTG ATT
Asn His Glu Leu Thr Gln Gln Leu Ala Gln Ile Asp Ala Glu Val Ile

CCC CTC AAT ATC CCG CTG CTG CTG CGT GAA GCC ATC GCC GAC CCC GCC
Pro Leu Asn Ile Pro Leu Leu Leu Arg Glu Ala Ile Ala Asp Pro Ala

CGT TAT GGC CTG GCA CTG GGG CAG GAC CTT GTC GCG ACC TGT TTC AAT
Arg Tyr Gly Leu Ala Leu Gly Gln Asp Leu Val Ala Thr Cys Phe Asn

GGC GAA GAG TGT ACG GAA AAC CCG CAG TAC GGA CTC AAC AGC GCA ACC

Gly Glu Glu Cys Thr Glu Asn Pro Gln Tyr Gly Leu Asn Ser Ala Thr

CCC AAC CCG GCC AAA CTG CTA TTC AAC GAC TCG GTA CAC CCC ACC GAG
Pro Asn Pro Ala Lys Leu Leu Phe Asn Asp Ser Val His Pro Thr Glu

ACA GGG CAA CAG CTG ATT GCC GAC TAT GCC TAC TCA TTG CTG GCC GTG
Thr Gly Gln Gln Leu Ile Ala Asp Tyr Ala Tyr Ser Leu Leu Ala Val

CCG TGG GAG CTG ACC TTG TTA CCC GTT ATG GCC CAA GGC TCG CTC AAC
Pro Trp Glu Leu Thr Leu Leu Pro Val Met Ala Gln Gly Ser Leu Asn

GCC CAT CAA GAC CAG TTG CGC AAC CAA TGG GCT GGC GAC GAC GGT CAA
Ala His Gln Asp Gln Leu Arg Asn Gln Trp Ala Gly Asp Asp Gly Gln

TGG CAA GCC ATT GGC CAG TGG CGA ACC CTG CTC GCC GGT GGC GGG CAA
Trp Gln Ala Ile Gly Gln Trp Arg Thr Leu Leu Ala Gly Gly Gly Gln

CGC CTG GAA ATC GAC AAG CAG ACC ACG GCC GTG AAG GCA GAT GGC AAA
Arg Leu Glu Ile Asp Lys Gln Thr Thr Ala Val Lys Ala Asp Gly Lys

GGC TAT AAC CTC AAT ATC GGC ACC AGC TAC AGG CTG GAT GAC AAC TGG
Gly Tyr Asn Leu Asn Ile Gly Thr Ser Tyr Arg Leu Asp Asp Asn Trp

CGA TTT GGC ATT GCC GGC GGG TTT TAC CGG CAA CGC CTG GAA ACC GGG
Arg Phe Gly Ile Ala Gly Gly Phe Tyr Arg Gln Arg Leu Glu Thr Gly

GCC AAT AAA TCG GAC TAC AAG CTC AAC AGC TAT CTG GGT AGC GTG TTT
Ala Asn Lys Ser Asp Tyr Lys Leu Asn Ser Tyr Leu Gly Ser Val Phe

GCC CAA TAC CAG CAC AAT CAC TGG TGG GGG GAT GCG GCA CTG ACG TTG
Ala Gln Tyr Gln His Asn His Trp Trp Gly Asp Ala Ala Leu Thr Leu

GGC AGG CTG GAT TAC GAC AGC CTG AAA CGA AAA TTC GCG CTT GGG GTG
Gly Arg Leu Asp Tyr Asp Ser Leu Lys Arg Lys Phe Ala Leu Gly Val

GGT AGT GAT ATG GAG CAA GGC CAG GCT GAC GGC CAT TTG CGA GCA TTG
Gly Ser Asp Met Glu Gln Gly Gln Ala Asp Gly His Leu Arg Ala Leu

GTA CAC GCC TGG GTT ATG AAA TCG CAC AGG CAT CTG ACC TGT GGC AGC
Val His Ala Trp Val Met Lys Ser His Arg His Leu Thr Cys Gly Ser

TCT CCC CCT TTA TCA GCG CCG TTA CTC GCG GGT GGA AGT TAA
Ser Pro Pro Leu Ser Ala Pro Leu Leu Ala Gly Gly Ser ***

## Claims

1. A gene coding a protein having a lipase activity originated from a bacterium of Pseudomonas genus, characterized by having the following base sequence:

10 20 30 40 50 60
5'ATGAAACATT TAATACAGTT TGTTTTGCTA ATGTCTGCGC CCCAAACAAC AAAAACAGGT
70 80 90 100 110 120
CATCACATGC GCAGGCTCTC CCTACTCATC CCTTGGCGGG GATGCATCCT GTCCATCGTC
130 140 150 160 170 180
AGTGAACGTG CCATAGCAGC ACCCTCGCCG TATTCCACCA TGATCGTGTT CGGCGACAGC
190 200 210 220 230 240
CTGAGCGACT CGGGGCATTT TCCAGGGGTC GGCACAGGCA TGCGCTTCAC CAACCGCACC
250 260 270 280 290 300
GGGCCGACGT ACAGGGATTA CCGGGGGGAG GAATATGCTG CAGTAACGCC CACGCGGCTG
310 320 330 340 350 360
GGCACCCAAC TGGGGATCGC CCCCGCAGAC CTGCGCCCGT CCACTTCGCC GGGCAACTCT
370 380 390 400 410 420
CTTGCAGGGG AGCCCGACGG CAACAACTGG GCGGTCGGCG GCTATCGCAC AGACCAGATC
430 440 450 460 470 480
CTCAATTCCA TCAAGACCGA GTCAAAGGTC GCCATCCCGG ATGACTGGTC CTTCGTCGGC
490 500 510 520 530 540
GGCTACGTCC TGCGCAGCAA GCCTGGCTAC CTTGTGCAGA ACAGCTTCAC GGCTGACCCC
550 560 570 580 590 600
AAGGCGTTGT ATTTCATTTC AGGCGGCGGT AATGACTTTC TCCAAGGCAA GGTCACCAAC
610 620 630 640 650 660
CCCGCCGAGG CAGGCCAAGC CGCACAAAGG CTGGCGGCAA GCGCCCACAC CCTGCAACAG
670 680 690 700 710 720
GCAGGGGCAC GCTACATCAT GGTCTGGCTG CTGCCCGATC TGGGCCTGAC GCCAGCCGTT
730 740 750 760 770 780
TACGGTACAC CCACCCAGGC TGGCACCAGC GCCCTGAGCG CCCTGTTCAA TCATGAGCTA

790 800 810 820 830 840
ACCCAGCAAC TGGCGCAGAT AGACGCCGAA GTGATTCCCC TCAATATCCC GCTGCTGCTG
850 860 870 880 890 900
CGTGAAGCCA TCGCCGACCC CGCCCGTTAT GGCCTGGCAC TGGGGCAGGA CCTTGTCGCG
910 920 930 940 950 960
ACCTGTTTCA ATGGCGAAGA GTGTACGGAA AACCCGCAGT ACGGACTCAA CAGCGCAACC
970 980 990 1000 1010 1020
CCCAACCCGG CCAAACTGCT ATTCAACGAC TCGGTACACC GCACCGAGAC AGGGCAACAG
1030 1040 1050 1060 1070 1080
CTGATTGCCG ACTATGCCTA CTCATTGCTG GCCGTGCCGT GGGAGCTGAC CTTGTTACCC
1090 1100 1110 1120 1130 1140
GTTATGGCCC AAGGCTCGCT CAACGCCCAT CAAGACCAGT TGCGCAACCA ATGGGCTGGC
1150 1160 1170 1180 1190 1200
GACGACGGTC AATGGCAAGC CATTGGCCAG TGGCGAACCC TGCTCGCCGG TGGCGGGCAA
1210 1220 1230 1240 1250 1260
CGCCTGGAAA TCGACAAGCA GACCACGGCC GTGAAGGCAG ATGGCAAAGG CTATAACCTC
1270 1280 1290 1300 1310 1320
AATATCGGCA CCAGCTACAG GCTGGATGAC AACTGGCGAT TTGGCATTGC CGGCGGGTTT
1330 1340 1350 1360 1370 1380
TACCGGCAAC GCCTGGAAAC CGGGGCCAAT AAATCGGACT ACAAGCTCAA CAGCTATCTG
1390 1400 1410 1420 1430 1440
GGTAGCGTGT TTGCCCAATA CCAGCACAAT CACTGGTGGG GGGATGCGGC ACTGACGTTG
1450 1460 1470 1480 1490 1500
GGCAGGCTGG ATTACGACAG CCTGAAACGA AAATTCGCGC TTGGGGTGGG TAGTGATATG
1510 1520 1530 1540 1550 1560
GAGCAAGGCC AGGCTGACGG CCATTTGCGA GCATTGGTAC ACGCCTGGGT TATGAAATCG
1570 1580 1590 1600 1610 1620
CACAGGCATC TGACCTGTGG CAGCTCTCCC CCTTTATCAG CGCCGTTACT CGCGGGTGGA
AGTTAA 3'

2. A gene according to claim 1 wherein said bacterium of Pseudomonas genus is Pseuodmonas fragi IFO 120 49 strain.

3. A recombinant DNA capable of being replicated in a bacterium of Pseudomonas genus and containing the gene of claims 1 or 2 incorporated into a broad host range vector of Gram-negative bacteria.

4. A bacterium of Pseudomonas genus transformed by a recombinant DNA according to claim 3.

5. A process for producing a protein having a lipase activity, which process comprises cultivating a bacterium according to claim 4, to thereby produce a protein having a lipase activity in the culture medium, and recovering this protein from the culture medium.

## Patentansprüche

1. Gen, das für ein Protein mit Lipaseaktivität codiert, und das von einem Bakterium der Gattung Pseudomonas herstammt, dadurch **gekennzeichnet,** daß es die folgende Basensequenz hat:

10 20 30 40 50 60
5'ATGAAACATT TAATACAGTT TGTTTTGCTA ATGTCTGCGC CCCAAACAAC AAAAACAGGT
70 80 90 100 110 120
CATCACATGC GCAGGCTCTC CCTACTCATC CCTTGGCGGG GATGCATCCT GTCCATCGTC
130 140 150 160 170 180
AGTGAACGTG CCATAGCAGC ACCCTCGCCG TATTCCACCA TGATCGTGTT CGGCGACAGC
190 200 210 220 230 240
CTGAGCGACT CGGGGCATTT TCCAGGGGTC GGCACAGGCA TGCGCTTCAC CAACCGCACC
250 260 270 280 290 300
GGGCCGACGT ACAGGGATTA CCGGGGGGAG GAATATGCTG CAGTAACGCC CACGCGGCTG
310 320 330 340 350 360
GGCACCCAAC TGGGGATCGC CCCCGCAGAC CTGCGCCCGT CCACTTCGCC GGGCAACTCT
370 380 390 400 410 420
CTTGCAGGGG AGCCCGACGG CAACAACTGG GCGGTCGGCG GCTATCGCAC AGACCAGATC
430 440 450 460 470 480
CTCAATTCCA TCAAGACCGA GTCAAAGGTC GCCATCCCGG ATGACTGGTC CTTCGTCGGC
490 500 510 520 530 540
GGCTACGTCC TGCGCAGCAA GCCTGGCTAC CTTGTGCAGA ACAGCTTCAC GGCTGACCCC
550 560 570 580 590 600
AAGGCGTTGT ATTTCATTTC AGGCGGCGGT AATGACTTTC TCCAAGGCAA GGTCACCAAC
610 620 630 640 650 660
CCCGCCGAGG CAGGCCAAGC CGCACAAAGG CTGGCGGCAA GCGCCCACAC CCTGCAACAG
670 680 690 700 710 720
GCAGGGGCAC GCTACATCAT GGTCTGGCTG CTGCCCGATC TGGGCCTGAC GCCAGCCGTT
730 740 750 760 770 780
TACGGTACAC CCACCCAGGC TGGCACCAGC GCCCTGAGCG CCCTGTTCAA TCATGAGCTA

790 800 810 820 830 840
ACCCAGCAAC TGGCGCAGAT AGACGCCGAA GTGATTCCCC TCAATATCCC GCTGCTGCTG
850 860 870 880 890 900
CGTGAAGCCA TCGCCGACCC CGCCCGTTAT GGCCTGGCAC TGGGGCAGGA CCTTGTCGCG
910 920 930 940 950 960
ACCTGTTTCA ATGGCGAAGA GTGTACGGAA AACCCGCAGT ACGGACTCAA CAGCGCAACC
970 980 990 1000 1010 1020
CCCAACCCGG CCAAACTGCT ATTCAACGAC TCGGTACACC GCACCGAGAC AGGGCAACAG
1030 1040 1050 1060 1070 1080
CTGATTGCCG ACTATGCCTA CTCATTGCTG GCCGTGCCGT GGGAGCTGAC CTTGTTACCC
1090 1100 1110 1120 1130 1140
GTTATGGCCC AAGGCTCGCT CAACGCCCAT CAAGACCAGT TGCGCAACCA ATGGGCTGGC
1150 1160 1170 1180 1190 1200
GACGACGGTC AATGGCAAGC CATTGGCCAG TGGCGAACCC TGCTCGCCGG TGGCGGGCAA
1210 1220 1230 1240 1250 1260
CGCCTGGAAA TCGACAAGCA GACCACGGCC GTGAAGGCAG ATGGCAAAGG CTATAACCTC
1270 1280 1290 1300 1310 1320
AATATCGGCA CCAGCTACAG GCTGGATGAC AACTGGCGAT TTGGCATTGC CGGCGGGTTT
1330 1340 1350 1360 1370 1380
TACCGGCAAC GCCTGGAAAC CGGGGCCAAT AAATCGGACT ACAAGCTCAA CAGCTATCTG
1390 1400 1410 1420 1430 1440
GGTAGCGTGT TTGCCCAATA CCAGCACAAT CACTGGTGGG GGGATGCGGC ACTGACGTTG
1450 1460 1470 1480 1490 1500
GGCAGGCTGG ATTACGACAG CCTGAAACGA AAATTCGCGC TTGGGGTGGG TAGTGATATG
1510 1520 1530 1540 1550 1560
GAGCAAGGCC AGGCTGACGG CCATTTGCGA GCATTGGTAC ACGCCTGGGT TATGAAATCG
1570 1580 1590 1600 1610 1620
CACAGGCATC TGACCTGTGG CAGCTCTCCC CCTTTATCAG CGCCGTTACT CGCGGGTGGA
AGTTAA 3'

2. Gen nach Anspruch 1, dadurch **gekennzeichnet,** daß das Bakterium der Gattung Pseudomonas der Pseudomonas fragi IFO-12049-Stamm ist.

3. Rekombinante DNA, die in der Lage ist, in einem Bakterium der Gattung Pseudomonas repliziert zu werden, und die das Gen nach Anspruch 1 oder 2 in einem Vektor mit breitem Wirtsbereich für gramnegative Bakterien eingebaut enthält.

4. Bakterium der Gattung Pseudomonas, das mit einer rekombinanten DNA nach Anspruch 3 transformiert ist.

5. Verfahren zur Herstellung eines Proteins mit Lipaseaktivität, wobei das Verfahren das Kultivieren eines Bakteriums nach Anspruch 4, wodurch ein Protein mit Lipaseaktivität im Kulturmedium gebildet wird, und das Gewinnen dieses Proteins aus dem Kulturmedium umfaßt.

## Revendications

1. Gène codant une protéine ayant une activité lipasique provenant d'une bactérie du genre Pseudomonas, caractérisé en ce qu'il a la séquence de bases suivante :

10 20 30 40 50 60
5'ATGAAACATT TAATACAGTT TGTTTTGCTA ATGTCTGCGC CCCAAACAAC AAAAACAGGT
70 80 90 100 110 120
CATCACATGC GCAGGCTCTC CCTACTCATC CCTTGGCGGG GATGCATCCT GTCCATCGTC
130 140 150 160 170 180
AGTGAACGTG CCATAGCAGC ACCCTCGCCG TATTCCACCA TGATCGTGTT CGGCGACAGC
190 200 210 220 230 240
CTGAGCGACT CGGGGCATTT TCCAGGGGTC GGCACAGGCA TGCGCTTCAC CAACCGCACC
250 260 270 280 290 300
GGGCCGACGT ACAGGGATTA CCGGGGGGAG GAATATGCTG CAGTAACGCC CACGCGGCTG
310 320 330 340 350 360
GGCACCCAAC TGGGGATCGC CCCCGCAGAC CTGCGCCCGT CCACTTCGCC GGGCAACTCT
370 380 390 400 410 420
CTGCAGGGG AGCCCGACGG CAACAACTGG GCGGTCGGCG GCTATCGCAC AGACCAGATC
430 440 450 460 470 480
CTCAATTCCA TCAAGACCGA GTCAAAGGTC GCCATCCGG ATGACTGGTC CTTCGTCGGC
490 500 510 520 530 540
GGCTACGTCC TGCGCAGCAA GCCTGGCTAC CTTGTGCAGA ACAGCTTCAC GGCTGACCCC
550 560 570 580 590 600
AAGGCGTTGT ATTTCATTTC AGGCGGCGGT AATGACTTTC TCCAAGGAA GGTCACCAAC
610 620 630 640 650 660
CCCGCCGAGG CAGGCCAAGC CGCACAAAGG CTGGCGGCAA GCGCCCACAC CCTGCAACAG
670 680 690 700 710 720
GCAGGGGCAC GCTACATCAT GGTCTGGCTG CTGCCCGATC TGGGCCTGAC GCCAAGCCTT
730 740 750 760 770 780
TACGGTACAC CCACCCAGGC TGGCACCAGC GCCCTGAGCG CCCTGTTCAA TCATGAGCTA
790 800 810 820 830 840
ACCCAGCAAC TGGCGCAGAT AGACGCCGAA GTGATTCCCC TCAATATCCC GCTGCTGCTG

850 860 870 880 890 900
CGTGAAGCCA TCGCCGACCC CGCCCGTTAT GGCCTGGCAC TGGGGCAGGA CCTTGTCGCG
910 920 930 940 950 960
ACCTGTTTCA ATGGCGAAGA GTGTACGGAA AACCCGCAGT ACGGACTCAA CAGCGCAACC
970 980 990 1000 1010 1020
CCCAACCCGG CCAAACTGCT ATTCAACGAC TCGGTACACC GCACCGAGAC AGGGAACAG
1030 1040 1050 1060 1070 1080
CTGATTGCCG ACTATGCCTA CTCATTGCTG GCCGTGCCGT GGGAGCTGAC CTTGTTACCC
1090 1100 1110 1120 1130 1140
GTTATGGCCC AAGGCTCGCT CAACGCCCAT CAAGACCAGT TGCGCAACCA ATGGGCTGGC
1150 1160 1170 1180 1190 1200
GACGACGGTC ATGGCAAGC CATTGGCCAG TGGCGAACCC TGCTCGCCGG TGGCGGGCAA
1210 1220 1230 1240 1250 1260
CGCCTGGAAA TCGACAAGCA GACCACGGCC GTGAAGGCAG ATGGCAAAGG CTATAACCTC
1270 1280 1290 1300 1310 1320
AATATCGGCA CCAAGCTACAG GCTGGATGAC AACTGGCGAT TTGGCATTGC CGGCGGGTTT
1330 1340 1350 1360 1370 1380
TACCGGCAAC GCCTGGAAAC CGGGGCCAAT AAATCGGACT ACAAGCTCAA CAGCTATCTG
1390 1400 1410 1420 1430 1440
GGTAGCGTGT TTGCCCAATA CCAGCACAAT CACTGGTGGG GGGATGCGGC ACTGACGTTG
1450 1460 1470 1480 1490 1500
GGCAGGCTGG ATTACGACAG CCTGAAACGA AAATTCGCGC TTGGGGTGGG TAGTGATATG
1510 1520 1530 1540 1550 1560
GAGCAAGGCC AGGCTGACGG CCATTTGCGA GCATTGGTAC ACGCCTGGGT TATGAAATCG
1570 1580 1590 1600 1610 1620
CACAGGCATC TGACCTGTGG CAGCTCTCCC CCTTTATCAG CGCCGTTACT CGCGGGTGGA
AGTTA 3'

2. Gène selon la revendication 1, où ladite bactérie du genre Pseudomonas est la souche Pseudomonas fragi IFO 120 49.

3. ADN recombiné capable de se répliquer dans une bactérie du genre Pseudomonas et contenant le gène selon les revendications 1 ou 2 incorporé dans un vecteur à large plage d'hôtes de bactéries à Gram négatif.

4. Bactérie du genre Pseudomonas transformée par un ADN recombiné selon la revendication 3.

5. Procédé de production d'une protéine ayant une activité lipasique, lequel procédé comprend la culture d'une bactérie selon la revendication 4, pour ainsi produire une protéine ayant une activité lipasique dans le milieu de culture et récupérer cette protéine dans le milieu de culture.
